(19) Europäisches Patentamt
European Patent Office
Office européen des brevets

(11) **EP 1 800 744 A1**

(12) **EUROPÄISCHE PATENTANMELDUNG**
veröffentlicht nach Art. 158 Abs. 3 EPÜ

(43) Veröffentlichungstag:
**27.06.2007   Patentblatt 2007/26**

(51) Int Cl.:
***B01J 19/10*** *(2006.01)*

(21) Anmeldenummer: 05749509.5

(22) Anmeldetag: **23.05.2005**

(86) Internationale Anmeldenummer:
**PCT/RU2005/000279**

(87) Internationale Veröffentlichungsnummer:
**WO 2005/123244 (29.12.2005 Gazette 2005/52)**

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **16.06.2004   RU 2004118091**

(71) Anmelder:
• **Obschestvo S Ogranichennoi Otvetstvennostyu "Astor-s"**
**Vologda, 160019 (RU)**

• **Shestakov, Sergei Dmitrievich**
**Vologda 160031 (RU)**

(72) Erfinder: **SHESTAKOV, Sergei Dmitrievich**
**Vologda, 160031 (RU)**

(74) Vertreter: **Jeck, Anton**
**Patentanwalt,**
**Klingengasse 2/1**
**71665 Vaihingen/Enz (DE)**

(54) **VERFAHREN ZUR BEHANDLUNG VON FLÜSSIGKEITEN IN EINEM KAVITATIONSREAKTOR**

(57)    Die Erfindung betrifft ein Verfahren zur Behandlung von Flüssigkeiten in einem Kavitationsreaktor. Bei dem Verfahren wird eine innerhalb des Kavitationsreaktors enthaltene Flüssigkeit mit einer Kavitation auslösenden Schallwelle mit einer vorgegebenen mittleren Energiedichte beaufschlagt. Dabei wird eine gleichmäßige Verteilung der Kavitationsenergie durch ein vorgebbares Verhältnis der Abmessungen des Innenraums des Kavitationsreaktors erreicht. Der Schalldruckbereich der Schallwelle wird mindestens 4,4 mal größer als der statische Druck der Flüssigkeit innerhalb des Kavitationsreaktors eingestellt.

EP 1 800 744 A1

**Beschreibung**

Die Erfindung betrifft ein Verfahren zur Ultraschall-Kavitationsbehandlung von Flüssigkeiten.

Ultraschall-Kavitationsbehandlungen können beispielsweise eine Zerstörung und/oder Abtrennung und/oder Teilung verschiedener in einer Flüssigkeit enthaltener Substanzen, wie etwa eine Abtötung von Mikroorganismen, die beispielsweise in Form von Schwebepartikeln in der Flüssigkeit vorkommen können, und/oder einer Dissoziation der Flüssigkeitsmoleküle zum Ziel haben. Eine Ultraschall-Kavitationsbehandlung einer Flüssigkeit kann darüber hinaus beispielsweise zur Erhöhung der Dispersität und Homogenität der vorhandenen Phasen, zur Verstärkung und/oder Beschleunigung von in der Flüssigkeit ablaufenden chemischen Reaktionen, wie etwa der Synthese und/oder der Analyse und/oder von der Bildung neuer Verbindungen nachfolgender Reaktionen, sowie zur Bakteriolyse und Bakteriostase dienen. Der Ultraschall-Kavitationsbehandlung können Emulsionen, Suspensionen, kolloidale bzw. echte Lösungen, sowie Wasser und andere Flüssigkeiten unterzogen werden. Der Vorgang der Energieübertragung in einer Flüssigkeit wie etwa Wasser durch Kavitationseinwirkung hat einen "epithermischen" Charakter, welcher der hochenergetischen Chemie eigene Prozesse sicherstellt, bei denen Wasser während kurzer Zeit aus dem thermodynamischen Gleichgewicht gebracht wird. Der Vorgang der Kavitationseinwirkung erlaubt eine bestimmte Menge Energie in der Flüssigkeit fast ohne Aufheizung zu akkumulieren und nachher diese Energie bei Rückkehr zum Gleichgewichtszustand als Hydratationswärme abzugeben.

Bekannt ist ein erstes Verfahren zur Behandlung von Flüssigkeiten in einem Kavitationsreaktor, bei dem eine Flüssigkeit mit einer eine vorgegebene Behandlungszeit sicherstellenden Geschwindigkeit durch den Kavitationsreaktor geleitet wird. Im Kavitationsreaktor wird eine stehende akustische Halbwelle mit einer vorgegebenen mittleren Energiedichte erzeugt. Durch die Energie dieser Halbwelle wird die Flüssigkeit zur Kavitation mit einem entsprechenden Erosionsfaktor angeregt. Dabei soll der einstellbare Erosionsfaktor, der am Umfang des Strömungsquerschnitts sein Maximum einnimmt, innerhalb des Kavitationsreaktors einen Mittelwert [1] nicht unterschreiten. Die Einstellung des Erosionsfaktors erfolgt durch Wahl von Größenverhältnis und Form eines Lochs in einer Membran, welche ein Bestandteil des Innenraums des Reaktors ist.

Dadurch wird neben einer Einstellung des erforderlichen Verhältnisses des kinetischen zum potentiellen Anteil der Kavitationsenergie auch eine bestimmte Gleichmäßigkeit ihrer Verteilung im durch das Loch in der Membran beschränkten Strömungsquerschnitt erzielt. Also werden die Form und das Größenverhältnis des Lochs in der Membran schließlich je nach Schalldruckbereich der akustischen Halbwelle $P$ festgelegt, der proportional zur Amplitude $A$ der Halbwelle $P$ und der Quadratwurzel der vorgegebenen mittleren Energiedichte, den mittleren Erosionsfaktor und Art seiner Verteilungsfunktion [2] bestimmt.

Das bekannte Verfahren erlaubt keine zufriedenstellende Behandlung von Flüssigkeiten, wenn keine Strömung durch das Loch in der Membran vorliegt. Beim bekannten Verfahren ist eine gleichmäßige Verteilung des Kavitationsfeldes nur innerhalb der Grenzen des in seiner Größe und in seiner Form durch die Prozessgrößen vorgegebenen Lochs in der Membran sichergestellt. Hierdurch kann beispielsweise keine portionsweise Behandlung, im Sinne einer chargenweisen Behandlung, eines abgeschlossenen Volumens einer Flüssigkeit stattfinden. Bekanntermaßen verändert sich außerdem bei einer Änderung der Randbedingungen der Kavitationsbehandlung, sofern diese eine Änderung der mittleren Schallenergiedichte nach sich ziehen, der Mittelwert sowie die Verteilungsfunktion des Erosionsfaktors [2].

Dadurch ist das bekannte Verfahren nicht universell für verschiedene Anwendungsfälle sowie für verschiedene Zwecke von Kavitationsbehandlungen anwendbar. Um beim bekannten Verfahren in verschiedenen Anwendungsfällen eine gleichmäßige Verteilung des Kavitationsfeldes zu erhalten, sind für jeden Anwendungsfall Änderungen des Innenraums des Kavitationsreaktor, in dem die Kavitationsbehandlung abläuft, durch Änderung der Form und Größe des Lochs in der Membran erforderlich.

Ein zweites, bekanntes Verfahren zur Kavitationsbehandlung von Flüssigkeiten sieht vor, eine Flüssigkeit mit einer vorgegebenen Geschwindigkeit durch einen Kavitationsreaktor zu leiten. Die Flüssigkeit wird mit einer Schalleistung mit einer vorgegebenen mittleren Energiedichte beaufschlagt, wodurch in der Flüssigkeit Kavitation auftritt. Die Schalleistung verteilt sich dabei in der Flüssigkeit. Die Verteilung der Energiedichte der potentiellen Kavitationsenergie entspricht dabei einer mittleren quadratischen Abweichung von der mittleren Energiedichte, wobei eine vorgegebene Abweichung nicht überschritten wird. Die Verteilungsfunktion der Energiedichte der potentiellen Kavitationsenergie wird dabei durch die Wahl des Verhältnisses von Innenmaßen des iCavitationsreaktorgehäuses bestimmt [3].

Beim zweiten Verfahren wird eine gleichmäßige Behandlung der Flüssigkeit unter Berücksichtigung der bekannten Tatsache erzielt, dass eine möglichst gleichmäßige Verteilung der Energiedichte der potentiellen Kavitationsenergie im gesamten Innenraum des Kavitationsreaktors die Hauptrolle bei der Kavitationsbehandlung spielt [4], und nicht nur eine gleichmäßige Verteilung in einem einzelnen Querschnitt.

Das zweite bekannte Verfahren erlaubt die Flüssigkeit im Kavitationsreaktor gleichmäßig zu behandeln, sowohl in einem kontinuierlichen Prozess, bei durch den Kavitationsreaktor strömender Flüssigkeit, als auch chargen- oder portionsweise und ohne Mischen bei im Kavitationsreaktor ruhender oder stehender Flüssigkeit.

Gleichzeitig weist das zweite Verfahren den Nachteil auf, dass die Verteilungsfunktion der Energiedichte der potentiellen Kavitationsenergie sowie die Verteilungsfunktion des Erosionsfaktors vom Schalldruckbereich der Schallwelle $P$ abhängt, dessen Quadrat proportional zur vorgegebenen mittleren Energiedichte der im Kavitationsreaktor verteilten Schallleistung ist.

Dies kann wie folgt nachgewiesen werden:

In der Theorie des Kavitationsreaktors [2], die als Grundlage bei der Berechnung der Größe des Gehäuses bzw. des Innenraums des Kavitationsreaktors dient, wird die Verteilung der potentiellen Kavitationsenergie im Raum durch das Integral des Quadrats der stückweise linearen verallgemeinerten Funktion

$$\delta(t,\phi) = [t + \phi] - t, \qquad\qquad (1)$$

über der Zeit beschrieben. Dabei bezeichnen $t, \phi$ den in Anteilen der Periode von der die Kavitation auslösenden Schallwelle ausgedrückten dimensionslosen Momentanwert der Zeit sowie die Startphase der Zeitmessung. [ ] bezeichnet den Betrag.

Mit dieser Funktion wird die so genannte dimensionslose Härte $\square i$ der Kavitationsblase näherungsweise bestimmt, d. h. eine Größe, die die Elastizität der Kavitationsblase charakterisiert.

Die Nutzung dieser Näherung ist jedoch nur dann zulässig, wenn die Kollapsphase der Kevitationsblasen - der Zeitabschnitt, während dem der momentane Radius R der Kavitationsblasen kleiner als deren Ruheradius $R_0$ ist - vernachlässigbar klein im Verhältnis zur Periode der die Kavitation auslösenden Schallwelle ist. Wenn diese Bedingung nicht erfüllt ist bzw. wenn die Blasen während einer Periode der Schallwelle mehrmals kollabieren, unterscheidet sich die Funktion $\delta(\tau,\phi)$ von der in (1) angegebenen Funktion partial linear. Dies ist bei niedrigen Werten des Schalldruckbereichs von der die Kavitation auslösenden Schallwelle der Fall, was eine Änderung der Verteilungsfunktion der Energiedichte der potentiellen Kavitationsenergie nach sich zieht.

Durch Integration z. B. der Hickling-Plesset-Differentialgleichung [5] für die Bewegung der Kavitationsblasenwände kann gezeigt werden, dass in einem Schalldruckbereich P, der z. B. 75 % des Ruhedrucks $P_0$ in der Kavitationsblase beträgt, die dimensionslose Härte $\square i$ der Kavitationsblase mit genügender Genauigkeit mit der sich von (1) wesentlich unterscheidenden Funktion

$$\delta(t,\phi) = -a \sin(\pi\phi) \sin[\pi(\phi + 2t)], \qquad\qquad (2)$$

näherungsweise bestimmt wird. Im betrachteten Fall mit $P = 0{,}75 P_0$ hat die Konstante $a$ den Wert $a = 0{,}28$.

Der Ausdruck für die Funktion $f$, mit der gemäß dem zweiten Verfahren die mittlere quadratische Abweichung der Energiedichte der potentiellen Kavitationsenergie von der mittleren Energiedichte berechnet wird, lautet:

$$f = \lambda^2 \left\{ \left(\frac{f_1}{\lambda} + 1\right)\left[\frac{f_1}{\lambda}\right] + \left[\frac{f_1}{\lambda} + 1\right]\frac{f_1}{\lambda} - \left[\frac{f_1}{\lambda}\right]^2 - \frac{f_1^2}{\lambda^2} - 2\left[\frac{f_1}{\lambda}\right]\right\}\frac{f_2^2}{f_1^2} \qquad (3)$$

Hierbei ist $\lambda$ die Länge der ausgestrahlten Schallwelle in der behandelten Flüssigkeit und $V$ das Volumen der an den Ultraschallgenerator angrenzenden Halbwellen-Resonanzzelle. Darüber hinaus steht $f_1$ für eine Funtkion, die den durchschnittlichen Abstand jedes beliebigen Punkts innerhalb des Volumens $V$ zum darin liegenden stationären Kavitationsgebiet angibt, welches im Betrieb des Kavitationsreaktors in einem Abstand von 0,25 $\lambda$ von der Oberfläche des Ultraschallgenerators entsteht. Darüber hinaus steht $f_2$ für eine Funktion, die einen durchschnittlichen Wert angibt, der umgekehrt proportional zum Abstand jedes beliebigen Punkts innerhalb des Volumens $V$ zum darin liegenden stationären Kavitationsgebiet ist, welches im Betrieb des Kavitationsreaktors in einem Abstand von 0,25 $\lambda$ von der Oberfläche des Ultraschallgenerators entsteht.

Durch Ersetzen der Näherungsfunktion (1) durch (2), was wie oben gezeigt bei niedrigen Schalldruckwerten $P$ notwendig ist, nimmt der Ausdruck (3) für $f$ dementsprechend folgende Form an:

$$f = \frac{1}{2} a^2 f_2^2 \sin^2\left(\frac{\pi}{\lambda} f_1\right) \qquad (4)$$

Durch Berechnung der mittleren quadratischen Abweichung der Energiedichte der potentiellen Kavitationsenergie von der mittleren Energiedichte, wie sie zur Beschreibung des zweiten Verfahrens verwendet wird, kann Folgendes gezeigt werden: bei einem Halbwellen-Kavitationsreaktor mit rundem Querschnitt mit einem Radius von z.B. 0,855 λ beträgt das Ergebnis der Berechnung gemäss Formel (3) 0,862 (obere Abweichungsgrenze des Prototyps), gemäss Formel (4) = 0,904. Anders gesagt, wird der Wert der oberen Abweichungsgrenze im letzten Fall bei einem Reaktorradius von 0,840 λ, und nicht bei 0,855 λ sichergestellt.

All das zeigt, dass auch bei dem zweiten bekannten Verfahren zur gleichmäßigen Behandlung der selben Flüssigkeit im Kavitationsreaktor bei verschiedenen Anwendungsfällen und Zwecken von Kavitationsbehandlungen mit dementsprechend verschiedenen Schalldruckwerten $P$ Änderungen des Innenraums des Kavitationsreaktors erforderlich sind.

Der Grundgedanke der Erfindung besteht im Folgenden.

Die oben angeführten Angaben, die auf allgemein zugänglichen Kenntnissen beruhen, beweisen, dass die Verteilungsfunktion der Energiedichte der potentiellen Kavitationsenergie von der durchschnittlichen Energiedichte der Schallleistung, die im Reaktor verteilt wird, und folglich auch vom Schalldruck $P$ der Schallwelle abhängt. Also kann ein Wertebereich für den Schalldruck $P$ bestimmt werden, bei dem eine solche Abhängigkeit vernachlässigbar klein ist. Ein in diesem vorher unbekannten Bereich ablaufendes Verfahren zur Behandlung von Flüssigkeiten in einem Kavitationsreaktor wird immer das gewünschte technische Ergebnis liefern.

Es ist bekannt, dass der Ruhedruck in einer Kavitationsblase vom Radius der Kavitationsblase, der Oberflächenspannung der Flüssigkeit, des Dampfdrucks der Flüssigkeit und dem auch als statischem Druck bezeichneten hydrostatischen Druck $P_h$ in der Flüssigkeit abhängt [2, 5]. Die reale Kavitationsschwelle in einer Flüssigkeit beträgt für Kavitationsblasen mit Durchmesser $2R_0$ von über $10^{-6}$ m bei einem hydrostatischen Druck von $10^5$ Pa weniger als $10^5$ Pa [6, 7], was sich durch einen niedrigen Dampfdruck im Vergleich zum hydrostatischen Druck im Gleichgewichtszustand realer Flüssigkeiten erklären lässt. Hieraus und mit Hilfe der aus [2, 5] bekannten Gleichung zur Berechnung des Gleichgewichts im Kavitationsblasenkeim kann nachgewiesen werden, dass sich der Ruhedruck $P_0$ in der Kavitationsblase im Gleichgewichtszustand von $R_0 > 10^{-5}$ m an bei verschiedenen Parametern vernachlässigbar wenig vom hydrostatischen Druck unterscheidet. Bei Vorgabe eines Schalldruckbereichs $P$ der Schallwelle, bei dem die Dichteverteilung der potentiellen Energie im Kavitationsreaktor invariant hierzu ist, kann man also den hydrostatischen Druck $P_h$ in der Flüssigkeit als Konstante annehmen, die als Anhaltspunkt zur Bestimmung des Schalldrucks $P$ dient.

Die Gleichungen für die Bewegung der Kavitationsblasenwände unter Einfluss des Außendrucks beinhalten als Parameter die Konstanten $P^*$ für den Innendruck und γ aus der Zustandsgleichung für eine Flüssigkeit vom Thetatyp [5]. Es ist bekannt [8], dass die Werte dieser Konstanten bei realen Flüssigkeiten innerhalb der Bereiche $P^* = 10^8...10^9$ Pa bzw. γ = 4...12 variieren.

Mit Hilfe der oben erwähnten Hickling-Plesset-Gleichung für die Bewegung der Kavitationsblasenwände sowie unter Berücksichtigung der Tatsache, dass sich die Schallgeschwindigkeit, Kompressibilität und Dichte vieler Flüssigkeiten und Lösungen innerhalb der technisch erreichbaren Grenzen für den hydrostatischen Druck $P_h$ [9] nur gering verändern, kann unter der Voraussetzung, dass ein Kollaps nicht mehr als zweimal pro Periode der die Kavitation auslösenden Schallwelle erfolgt, gezeigt werden, dass der Streubereich der Zeitpunkte des Kollapsbeginns und die Abweichung der Funktion □$i$ von (1) bei einer Variation von $P^*$ und γ in den oben angegebenen Bereichen gering sind. Je größer $P$ ist, desto kürzer die Kollapsdauer. Für alle realen Flüssigkeiten im Bereich der technisch erreichbaren Werte für den hydrostatischen Druck $P_h$ wird die dimensionslose Härte □$i$ der Kavitationsblase mit genügender Genauigkeit mit der Funktion (1) für alle beliebigen Werte des Schalldrucks $P$, bei dem der Kollaps nicht öfter als 2 mal pro Periode der die Kavitation auslösenden Schallwelle erfolgt, beschrieben. Diese Behauptung kann auch anhand des Druckstoßerhaltungssatzes überprüft werden, denn unabhängig davon, ob das Verhältnis der Pausen zwischen aufeinander folgenden Kollapsen der einzelnen Kavitationsblasen zu den Perioden der Schallwelle [2] gerade oder ungerade ist, ist der vom Kavitationsgebiet in einer größeren Periodenzahl erzeugte Durchschnittsdruck gleich Null. Das bedeutet, dass die Verteilung der potentiellen Energie im Kavitationsreaktor dabei invariant zum Schalldruck $P$ ist.

Der gesuchte Wert für den Schalldruck $P$, der durch numerische Integration mit Hilfe der Rekursivprozedur aus der Hickling-Plesset-Gleichung [2] erhalten wird, die gemäß den oben angeführten Überlegungen durch Computersimulationen und analytische Berechnungen verifiziert wurde, beträgt 4,4 $P_h$.

Das mit einem Schalldruck $P$, der das 4,4-fache des hydrostatischen Drucks $P_h$ im Kavitationsreaktor beträgt, erzielte technische Ergebnis ist die Sicherstellung einer gleichmäßigen Einwirkung von potentieller Kavitationsenergie auf eine im Kavitationseraktor befindliche zu behandelnde Flüssigkeit. Durch das erfindungsgemäße Verfahren wird bei vorgegebenen Abmessungen des Kavitationsreaktors für verschiedene Anwendungsfälle und Zwecke des Behandlungsprozesses eine gleichmäßige Einwirkung erreicht, sowohl bei der kontinuierlichen Durchlauf als auch bei der chargenweisen Portionsbehandlung.

Bei einer Ausführung des erfindungsgemäßen Verfahrens wird das angegebene technische Ergebnis dadurch erzielt, dass der eingestellte Schalldruckbereich $P$ der mit vorgegebener mittlerer Energiedichte innerhalb des Kavitationsreaktors verteilten, die Kavitation auslösenden Schallwelle mindestens 4,4 mal größer als der statische Druck $P_h$ in der Flüssigkeit innerhalb des Kavitationsreaktors ist. Demgegenüber wird bei den aus dem Stand der Technik bekannten Verfahren zur Behandlung von Flüssigkeiten im Kavitationsreaktor eine gleichmäßige Verteilung der Kavitationsenergie durch eine entsprechende Wahl der Größe der Innenabmessungen des Kavitationsreaktors erreicht.

Das erfindungsgemäße Verfahren kann beispielsweise zur selektiven Entkeimung von Flüssigkeiten, z. B. von Milch, verwendet werden, etwa um die Milchsäuremikroflora zu erhalten und krankheiterregende Mikroorganismen zu eliminieren.

Eine mögliche Aufgabenstellung könnte beispielsweise lauten, das Verhältnis der Abmessungen eines Kleinkavitationsreaktor mit rundem Querschnitt festzulegen, der mit einer Frequenz von 20 kHz mit einer maximalen Schallleistung von 750 W betrieben wird. In dem Kavitationsreaktor soll Kuhvollmilch sowohl einer vollständigen Bakteriolyse als auch einer selektiven Entkeimung von der Bakteriengruppe *Escherichia coli* unterzogen werden können. Dabei soll die Behandlung in beiden Fällen unter den bekannten optimalen Betriebsarten und unter der Bedingung einer größtmöglich gleichmäßigen Behandlung der in den Kavitationsreaktor eingebrachten Flüssigkeit ablaufen.

Es ist bekannt [2], dass zur vollständigen Bakteriolyse die optimale Energiedichte einer Kavitation in Milch auslösenden Schallwelle 1,5 W/cm$^3$ beträgt. Ebenso ist bekannt, dass die optimale Energiedichte zur Eliminierung von Kolibakterien 0,8 W/cm$^3$ beträgt. Das Reaktorvolumen soll also 750 : 1,5 = 500 cm$^3$ betragen, um beide Forderungen erfüllen zu können.

Wenn die Reaktorhöhe und die Länge der Halbwelle, welche für Milch ca. 3,5 cm beträgt, gleich sind, wird die Bedingung einer gleichmäßigen Kavitationsbehandlung gemäß dem zweiten bekannten Verfahren nicht erfüllt. Bei einem Radius von $\sqrt{500 : 3,5\pi} = 6,7$ cm geht in diesem Fall die mittlere quadratische Abweichung der Energiedichte der potentiellen Kavitationsenergie von der mittleren Energiedichte über 0,862 hinaus und beträgt 1,057. Um die Forderung der Gleichmäßigkeit zu erfüllen, müsste der Reaktor demnach eine der Wellenlänge gleiche Höhe von ca. 7 cm haben. Sein Radius würde dabei 4,8 cm und die mittlere quadratische Abweichung der Energiedichte der potentiellen Kavitationsenergie von der mittleren Energiedichte würde dementsprechend 0,686 betragen. Hierbei wird aber für die vollständige Bakteriolyse und für die Eliminierung der Kolibakterien nicht die Gleichmäßigkeit erreicht, da der Schalldruckbereich der Schallwelle gleich $\sqrt{2Ic\rho}$ ist. Dabei ist c die Schallgeschwindigkeit in Milch und ρ bezeichnet ihre Dichte. / gibt die in dieser Periode durchschnittliche Intensität der Schallwelle an, welche sich aus dem Verhältnis der eingebrachten Schalleistung zur Querschnittsfläche des Kavitationsreaktors ergibt. I beträgt bei der ersten Anwendung 5,42 Ph, in der zweiten 3,96 $P_h$, was weniger als 4,4 $P_h$ ist.

Ohne das erfindungsgemäße Verfahren in Betracht zu ziehen, kann demzufolge eine gleichmäßige bakteriziden Behandlung von Milch in den geschilderten Anwendungsfällen und zu den genannten Zwecken nicht erreicht werden.

Wird das erfindungsgemäße Verfahren jedoch in Betracht gezogen, sollte eine Kavitationsreaktorhöhe von 1,5 λ gewählt werden. Dann beträgt die mittlere quadratische Abweichung der Energiedichte der potentiellen Kavitationsenergie von der mittleren Energiedichte 0,629. Der Schalldruckbereich der Schallwelle für die gesamte Bakteriolyse ist in diesem Fall gleich 6,64 $P_h$ und für die Eliminierung der Kolibakterien gleich 4,85 $P_h$. D. h. die Bedingung der Gleichmäßigkeit wird mit dem erfindungsgemäßen Verfahren erfüllt.

Bevorzugtes Ausführungsbeispiel des erfindungsgemäßen Verfahrens.

Vollmilch soll zur Desinfektion von Kolibakterien in einem Kavitationsreaktor mit rundem Querschnitt mit einer Frequenz 20 kHz kontinuierlich behandelt werden. Der Prozessdurchsatz soll dabei mindestens 300 L/h betragen. Der hydrostatische Druck im Kavitationsreaktor entspricht dem Druck in der Milchleitung und beträgt 1,5 bar.

Es ist bekannt, dass die Behandlungszeit dabei 22,5 sec. und die Leistungsdichte der Schallwelle 0,8 W/cm$^3$ beträgt. Demzufolge soll das Kavitationsreaktorvolumen mindestens 300*1000*22,5:3600 = 1875 cm$^3$ betragen. Zur Vereinfachung wird von einem Kavitationsreaktorvolumen von 2 I ausgegangen. Die in den Kavitationsreaktor eingebrachte Schallleistung beträgt 0,8*200 = 1600 W.

Mit Hilfe des oben angeführten Ausdrucks für den Schalldruckbereich der Schallwelle, der gemäß dem Merkmal der Erfindung 4,4$P_h$ nicht unterschreiten darf, und der Wellenintensität, die dem Verhältnis der eingebrachten Schallleistung zur Querschnittsfläche des Kavitationsreaktors entspricht, kann die Querschnittsfläche des Kavitationsreaktors ermittelt werden. Sie beträgt 103 cm$^2$. Anhand der Schallwellenlänge in Milch, die bei der vorgegebenen Frequenz 7 cm beträgt, kann anhand der Querschnittsfläche des Kavitationsreaktors der Reaktorradius ermittelt werden. Er beträgt 5,5 cm, die Reaktorhöhe beläuft sich dabei auf drei Wellenlängen. Bei diesem Radius ist $P$ = 4,6$P$h>4,4$P$h, und die mittlere quadratische Abweichung der Energiedichte der potentiellen Kavitationsenergie von der mittleren Energiedichte beläuft sich auf 0,794<0,862, d. h. es werden die Forderungen erfüllt, die sowohl vom Stand der Technik, als auch vom erfindungsgemäßen Verfahren vorgegeben sind.

Da der Innenraum des Kavitationsreaktors zylinderförmig ist, kann die Leistungsdichte der Schallwelle und ihr berechneter Schalldruckbereich anhand des Schwingverschiebungsbereichs der Oberfläche des Ultraschallgenerators bestimmt werden, von dem aus sich die Schallwelle verbreitet, indem beispielsweise die Speisestromamplitude des elektroakustischen Ultraschallgenerators gemessen wird.

Amplitude der Oberfläche des Ultraschallgenerators, die nach bekannten Formeln berechnet wird, beträgt 3,9 $\mu$m und kann im realen Prozess durch bekannte technische Mittel kontrolliert werden [6].

Der nach dem beschriebenen Verfahren zur Berechnung der Verhältnisse der Kavitationsreaktorabmessungen hergestellte Kavitationsreaktor kann zur Behandlung anderer Flüssigkeiten in anderen Anwendungsfällen und zu anderen Zwecken der Behandlung verwendet werden, vorausgesetzt, dass die erforderliche Leistungsdichte der Schallwelle dabei mindestens 0,8 W/cm$^3$, der hydrostatische Druck der Flüssigkeit maximal 1,5 bar beträgt und der Prozessdurchsatz groß genug ist.

Dementsprechend zeugen die oben angeführten Angaben von der Möglichkeit einer Ausführung des erfindungsgemäßen Verfahrens mittels der oben beschriebenen oder vorher bekannten Mittel und Methoden sowie von der Erreichbarkeit des oben angegebenen technischen Ergebnisses bei Verwirklichung der gesamten Erfindungsmerkmale.

Die Erfindung kann in der Lebensmittel-, Chemie-, Pharma- und Parfümindustrie, in der Petrochemie, im Erdöl- und Erzbergbau sowie in der Medizin verwendet werden.

**Bibliographische Angaben**

[1] RU 2226428, 17.04.2003

[2] S.D.Schestakow: "Grundlagen der Technologie der Kavitationsdesintegration"; EWA-press Moskau 2001, 173 Seiten.

[3] RU 2228217, 21.05.2003

[4] L.Bergman: "Ultraschall und seine Anwendung in Wissenschaft und Technik"; IIL Moskau 1956, 726 Seiten.

[5] R.Knapp, J.Daily, F.Hammit: "Kavitation"; Mir, Moskau 1974, 348 Seiten.

[6] W.Mazon: "Physikalische Akustik. Verfahren und Geräte für Ultraschalluntersuchungen"; Mir Moskau, 1967, Band 1, Teil "B", 362 Seiten.

[7] W.A.Krassilnikow, W,W.Krylow: "Einführung in die physikalische Akustik"; Nauka Moskau 1984, 40a Seiten.

[8] L.K.Sarembo, W.I.Timoschenko: "Nichtlineare Akustik"; Verlag der Moskauer Universität. Moskau 1984, 104 Seiten

[9] W.Mazon: "Physikalische Akustik. Eigenschaften der Gase, Flüssigkeiten und Lösungen"; Mir Moskau 1968, Band 2, Teil "A", 488 Seiten

**Patentansprüche**

1. Verfahren zur Behandlung von Flüssigkeiten in einem Kavitationsreaktor, bei dem eine innerhalb des Kavitationsreaktors enthaltene Flüssigkeit mit einer Kavitation auslösenden Schallwelle mit einer vorgegebenen mittleren Energiedichte beaufschlagt wird, wobei eine gleichmäßige Verteilung der Kavitationsenergie durch ein vorgebbares Verhältnis der Abmessungen des Innenraums des Kavitationsreaktors erreicht wird,
**dadurch gekennzeichnet,**
**dass** der Schalldruckbereich der Schallwelle mindestens 4,4 mal größer als der statische Druck der Flüssigkeit innerhalb des Kavitationsreaktors eingestellt wird.

## INTERNATIONAL SEARCH REPORT

| International application No. |
|---|
| PCT/RU 2005/000279 |

**A.    CLASSIFICATION OF SUBJECT MATTER**

B01J 19/10

According to International Patent Classification (IPC) or to both national classification and IPC

**B.    FIELDS SEARCHED**

Minimum documentation searched (classification system followed by classification symbols)

B01J 19/00, 19/10, B01F 11/00, 11/02, C02F 1/36, C21C 5/48

Documentation searched other than minimum documentation to the extent that such documents are included in the fields searched

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| A | RU 2228217 C1 (SHESTAKOV SERGEI DMITRIEVICH), 10.05.2004, the abstract | 1 |
| A | SU 511354 A (INSTITUT CHERNOI METALLURGII), 05.07.1976, column 4, lines 15-23 | 1 |
| A | WO 1994/009894 A1 (KLADOV ANATOLY FEDOROVICH), 11.05.94, page 11, lines 1-6 | 1 |

☐  Further documents are listed in the continuation of Box C.          ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| "O" | document referring to an oral disclosure, use, exhibition or other means | "Y" | document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" | document member of the same patent family |

| Date of the actual completion of the international search | Date of mailing of the international search report |
|---|---|
| **17 August 2005 (17.08.2005)** | **01 September 2005 (01.09.2005)** |

| Name and mailing address of the ISA/ | Authorized officer |
|---|---|
| Facsimile No. | Telephone No. |

Form PCT/ISA/210 (second sheet) (July 1992)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- RU 2226428
- RU 2228217

### In der Beschreibung aufgeführte Nicht-Patentliteratur

- **S.D.SCHESTAKOW.** Grundlagen der Technologie der Kavitationsdesintegration. EWA-press, 2001, 173
- **L.BERGMAN.** Ultraschall und seine Anwendung in Wissenschaft und Technik. *IIL Moskau,* 1956, 726
- **R.KNAPP ; J.DAILY ; F.HAMMIT.** Kavitation. *Mir, Moskau,* 1974, 348
- **W.MAZON.** Physikalische Akustik. Verfahren und Geräte für Ultraschalluntersuchungen. *Mir Moskau,* 1967, vol. 1, 362
- **W.A.KRASSILNIKOW ; W,W.KRYLOW.** Einführung in die physikalische Akustik. *Nauka Moskau,* 1984, 40a
- **L.K.SAREMBO ; W.I.TIMOSCHENKO.** Nichtlineare Akustik. Verlag der Moskauer Universität, 1984, 104
- **W.MAZON.** Physikalische Akustik. Eigenschaften der Gase, Flüssigkeiten und Lösungen. *Mir Moskau,* 1968, vol. 2, 488